# NEW EUROPEAN PATENT SPECIFICATION

(11) **EP 3 355 894 B2**
(45) Date of publication and mention of the opposition decision: **04.11.2026**
(45) Mention of the grant of the patent: 24.04.2024
(21) Application number: 15905564.9
(22) Date of filing: 29.09.2015
(51) Int. Cl.: A61K 31/7016, A61K 31/719, A61K 31/702, A61K 31/194, A61P 15/02, A61K 31/19, A61K 31/191, A61K 31/192, A61K 31/7004

(54) **COMPOSITION COMPRISING SUGAR FOR MAINTAINING LACTOBACILLUS DOMINANCE IN THE UROGENITAL AREA**
ZUSAMMENSETZUNG ENTHALTEND ZUCKER ZUR AUFRECHTERHALTUNG DER LACTOBACILLUS-DOMINANZ IM UROGENITALBEREICH
COMPOSITION AVEC DU SUCRE POUR LE MAINTIEN DE LA DOMINANCE DE LACTOBACILLUS DU TRACTUS UROGÉNITAL

(43) Date of publication of application: 08.08.2018
(60) Divisional application: 21150997.1 / 3 831 391
(73) Proprietor: Kimberly-Clark Worldwide, Inc., Neenah, Wisconsin 54956 (US)
(72) Inventor: LI, Jingru, Roswell, Georgia 30076 (US); PEED, Lindsay, Adrienne, Roswell, Georgia 30076 (US); JOYNER, Cheryce, Francina, O'fallon, IL 62269 (US); VONGSA, Rebecca, Ann, Neenah, Wisconsin 54956 (US); KOENIG, David, William, Neenah, Wisconsin 54956 (US); BARTELL, Ryan, Daniel, Neenah, Wisconsin 54956 (US)
(74) Representative: Dehns
(86) International application number: PCT/US2015/052951
(87) International publication number: WO 2017/058174

(56) References cited:
- EP-A1- 1 911 454
- EP-A1- 1 946 760
- WO-A1-2006/053170
- WO-A1-2015/135470
- WO-A1-97/29763
- CA-A1- 2 942 424
- CN-A- 1 562 049
- CN-A- 101 411 714
- CN-A- 102 870 987
- US-A1- 2006 105 963
- US-A1- 2012 201 796
- US-B2- 6 964 949
- O. IBRAHIM OSAMA, IBRAHIM OSAMA O: "Functional Oligosaccharide: Chemicals Structure, Manufacturing, Health Benefits, Applications and Regulations", JOURNAL OF FOOD CHEMISTRY & NANOTECHNOLOGY, vol. 04, no. 04, 1 January 2018 (2018-01-01), pages 65 - 76, XP055938196, DOI: 10.17756/jfcn.2018-060
- CHANG, C. E. ET AL.: "Cultivation of Lactobacillus crispatus KLB46 isolated from human vagina", BIOTECHNOLOGY AND BIOPROCESS ENGINEERING, vol. 6, no. 2, 2001, pages 128 - 132, XP055385957

## Description

### BACKGROUND OF THE DISCLOSURE

Humans are colonized by microbes in the gastrointestinal tract, on the skin, and in other epithelial and tissue niches such as the oral cavity, eye surface and vagina. In healthy persons a single local or tissue type may be inhabited by hundreds of different species of bacteria. Interactions between various bacteria species in these populations and between bacteria and the human host shape the community structure with availability of and competition for resources affecting the distribution of various species of bacteria. Such resources may be food, location and the availability of space to grow or a physical structure to which the bacteria may attach.

A healthy microbial flora provides the host with multiple benefits, including colonization resistance to a broad spectrum of pathogens, essential nutrient biosynthesis and absorption, and immune stimulation. For example, a normal vagina generally contains more than about 10⁴ lactobacilli per milliliter of vaginal fluid. Under normal conditions, the vagina flora provides a mildly acidic environment that helps guard against the invasion of pathogenic microbes. Unfortunately, this vaginal balance may be easily upset by a variety of external factors that ultimately lead to vaginal infection. Vaginal infection is a clinical syndrome and exists in three primary forms, i.e., bacterial vaginosis, candidal vaginitis ("yeast"), and trichomonas vaginitis ("trich").

Current treatment regimens for bacterial infection of the vagina involve the use of various broad spectrum antibiotics, such as metronidazole. However, antibiotics are often undesirable because they may kill a broad range of the normal bacterial flora in the vagina, including the beneficial lactobacilli. This may cause secondary complications, because the lactobacilli keep various opportunistic pathogens in the vagina in check. The treatment may then necessitate a further treatment regimen, such as the ingestion of cultured dairy products to replace the lactobacilli in the body, as well as treatment by antifungal agents. Moreover, a rise in the level of anaerobes due to a lack of lactobacilli could further complicate the infection. Additionally, antibiotics, when used frequently within the vagina, may cause systemic toxicity through absorption from the vagina.

As such, a need currently exists for improved compositions for supporting and maintaining a healthy balance of microflora in the urogenital area and more particularly improved compositions.

WO 2015/135470 discloses the use of isomaltulose in the preparation of a vaginal composition.

EP 1 911 454 discloses the use of benzoic acid and/or its sodium salt in combination with saccharide(s) as active components for preparing a vaginal composition for modulating vaginal bacterial flora and vaginal acidity.

WO 2006/053170 discloses a vaginal treatment composition that employs a therapeutic agent to inhibit and/or treat vaginal infection.

### SUMMARY OF THE DISCLOSURE

It has now been surprisingly discovered that the growth of certain strains of *Lactobacilli* may be increased by administering a pharmaceutical composition comprising a single therapeutic agent selected from the group consisting of maltitol, pullulan, maltotriose, 2-deoxy-D-ribose, 1-kestose and erlose. In certain embodiments increasing the growth of beneficial lactobacilli may effectively inhibit the growth of pathogens associated with urogenital infections and help maintain a healthy microflora balance in the urogenital area. As such compositions comprising a single therapeutic agent are well suited for topical administration to the urogenital area of a female for support and maintain a healthy balance of microflora in the urogenital area. For example, providing a composition comprising a single therapeutic agent selected from the group consisting of maltitol, pullulan, maltotriose, 2-deoxy-D-ribose, 1-kestose and erlose promotes the growth of lactobacilli without promoting growth of pathogenic bacteria such as *Escherichia coli* or *Gardnerella vaginalis.*

Accordingly, the present invention provides a composition for use in maintaining a healthy microflora balance in the urogenital area of a patient in need thereof by topically administering the composition to the urogenital area of a patient, the composition comprising a single therapeutic agent selected from the group consisting of maltitol, pullulan, maltotriose, 2-deoxy-D-ribose, 1-kestose and erlose, and an aqueous solvent, wherein the urogenital area is the vulva, vagina, or urinary tract. In a particularly preferred embodiment administration of the pharmaceutical composition not only promotes the growth of lactobacilli but also results in the inhibition of enteropathogenic bacteria, such as *Gardnerella vaginalis.*

The pharmaceutical compositions of the present invention may be formulated into formulations for administration of a user in need thereof. Suitable formulations may include, for example, liquids, solutions, pastes or gels. Accordingly, in one preferred embodiment the present invention provides a formulation for topical administration to a user comprising a single therapeutic agent selected from the group consisting of maltitol, pullulan, maltotriose, 2-deoxy-D-ribose, 1-kestose and erlose and from about 0.05 to about 5.0 wt/vol % of at least one gelling agent that includes gellan gum. In a particularly preferred embodiment the single therapeutic agent comprises from about 0.1 to about 2.0 wt/vol % and the pH of the formulation is from about 3.5 to about 5.5.

The compositions of the present invention may be applied to an applicator. Suitable applicators include a web, such as a wet laid tissue web or air laid web, gauze, cotton swab, transdermal patch, container or holder. Thus, the composition may be applied to a nonwoven web, such as meltblown, coform, spunbond, airlaid, hydroentangled nonwovens, spunlace, bonded carded webs, and laminates thereof, as well as wet laid fibrous webs, such as tissue webs.

The compositions of the present invention may be administered to a user to provide a therapeutic effect.

### DEFINITIONS

As used herein, the term "inhibit" generally means to reduce by a measurable amount or to prevent entirely.

As used herein the term "urogenital" refers to the vulva, vagina, or urinary tract.

As used herein the terms "effective amount" and "therapeutic amount" is an amount sufficient to inactivate, but not necessarily kill, pathogenic microorganisms responsible for vaginal infection. In fact, although not required, it may be desired to use a concentration that does not significantly affect or inhibit the growth characteristics of the normal vaginal flora or otherwise significantly irritate the vaginal tissue when used at inhibitory, noncytotoxic, or clinical concentrations. For example, the single therapeutic agent is desirably employed at a concentration of about 0.01 to about 5.0 wt/vol %, in some embodiments from about 0.1 to about 2.0 wt/vol %, in some embodiments from about 0.2 to about 1.5 wt/vol %, and in some embodiments from about 0.5 to about 1.0 wt/vol %. It should be understood that the dosage may vary with the age, condition, and type of infection suffered by the patient, and may be readily determined by one of skill in the art.

As used herein the term "therapeutic effect" refers to the ability of the compositions and formulations of the present invention to stimulate the growth of *L. crispatus* relative *E. coli* measured according to the therapeutic effect protocol described below. Generally therapeutic effect is expressed as a ratio of *L. crispatus* to *E. coli* and is desirably greater than about 30, more preferably greater than about 50 and more desirably greater than about 100.

As used herein, the designation "wt/vol %" or "wt/vol" refers to the value obtained by dividing the weight of a substance (in grams) by the volume of the solution (in milliliters), and then multiplying by 100.

As used herein the term "soluble" when having reference to a pentose, a disaccharide, an organic acid, a cyclodextrin, a pectic substance or a non-digestible polysaccharide means that the substance is at least soluble according to the method described by L. Prosky et al, J. Assoc. Off. Anal. Chem. 71, 1017-1023 (1988).

### DETAILED DESCRIPTION OF THE DISLOSURE

The compositions and formulations of the present invention are intended to stimulate the growth of Gram-positive rod-shaped bacteria belonging to the species *Lactobacilli spp.* It is believed that stimulating the growth of, and dominance of, lactobacillus reestablishes healthy flora by reducing or excluding the population of pathogenic bacteria. The compositions of the present invention comprise only a single therapeutic agent capable of facilitating the growth of Gram-positive rod-shaped bacteria belonging to the species *Lactobacilli spp.* The single therapeutic agent is selected from the group consisting of maltitol, pullulan, maltotriose, 2-deoxy-D-ribose, 1-kestose and erlose.

Compositions and formulations of the present invention are useful in supporting and maintaining healthy microflora balance in the urogenital area. For example, maintenance and support of a healthy microflora may be achieved by topically administering a composition to the urogenital tract.

The therapeutic composition comprises only a single therapeutic agent selected from the group consisting of maltitol, pullulan, maltotriose, 2-deoxy-D-ribose, 1-kestose and erlose. The composition also includes an aqueous solvent and may include other components such as, for example, a stabilizer, a surfactant, a solubilizer, a buffer, a suspending agent, a colorant, a pH adjuster, a viscosity increasing agent, a dispersant, or a preservative, but only comprises one therapeutic agent capable of providing a therapeutic effect.

Urogenital treatment compositions of the present invention generally stimulate the growth of healthy, native, bacteria such as *Lactobacillus spp.* and may be administered in several forms to a user. For example, the urogenital compositions may be prepared as formulations for administration to a user or may be applied to a substrate, such as a wiping substrate, for administration to a user. Preferably the saccharides useful in the present invention are soluble to facilitate their formulation for administration to a user.

Surprisingly compositions comprising a single therapeutic agent selected from the group consisting of maltitol, pullulan, maltotriose, 2-deoxy-D-ribose, 1-kestose and erlose promote the growth of healthy bacteria such as *Lactobacillus spp.* and more particularly *Lactobacillus acidophilus* without promoting growth of enteropathogenic bacteria, such as *Gardnerella* (e.g., *Gardnerella vaginalis*), *Candida* (e.g., *Candida albicans*), and/or *Trichomonas* (e.g., *Trichomonas vaginalis*). Accordingly, compositions of the present invention may be administered to a user to selectively stimulate growth of lactobacilli without stimulating the growth of competing enteropathogenic bacteria. Thus, in-use, administration of a formulation comprising a single therapeutic agent selected from the group consisting of maltitol, pullulan, maltotriose, 2-deoxy-D-ribose, 1-kestose and erlose may enhance the growth and colonization of healthy bacteria such as *Lactobacillus spp.* in the user, which thereby helps reduce the incidence of disease.

Accordingly, the composition comprising a single therapeutic agent selected from the group consisting of maltitol, pullulan, maltotriose, 2-deoxy-D-ribose, 1-kestose and erlose may affect the growth of *L. crispatus* over *E. coli,* as measured using the therapeutic effect protocol described below. Preferably the composition yields a ratio of *L. crispatus* to *E. coli* greater than about 30, still more preferably greater than about 50 and still more preferably greater than 100, and even more preferably greater than about 300.

Compositions useful in the present invention comprise only a single therapeutic agent. The therapeutic agent generally comprises a saccharide. For example, in one embodiment, the composition may comprise 2-deoxy-D-ribose. In other embodiments the single therapeutic agent may be maltitol, which has a formula of C₁₂H₂₄O₁₁. In yet other embodiments the single therapeutic may be pullulan, which is composed of maltotriose units and has then general formula (C₆H₁₂O₅)ₙ. In still other embodiments the single therapeutic agent may be maltotriose, 1-kestose or erlose.

The composition comprises only a single therapeutic agent. That is to say that the composition generally comprises only one therapeutic agent selected from the group of maltitol, pullulan, maltotriose, 2-deoxy-D-ribose, 1-kestose and erlose. Other agents known to be carbon sources for gram-positive bacteria and have a therapeutic effect such as glucose, fructose, galactose, mannose, lactose, lactulose, mycose, cellobiose, melibiose, melitose, dextrin, starch and glycogen are not components of the present composition. Surprisingly compositions comprising only a single therapeutic agent have been shown to stimulate the growth of lactobacillus and maintain healthy flora without the addition of a second carbon source. As such compositions of the present invention generally comprise only a single carbon source that may be utilized by gram negative bacteria and it is preferred that the single carbon source be selected from the group of maltitol, pullulan, maltotriose, 2-deoxy-D-ribose, 1-kestose and erlose.

Generally compositions of the present invention comprise less than about 10.0 wt/vol % therapeutic agent. In particularly preferred embodiments the total amount of therapeutic agent is less than about 5.0 wt/vol % and still more preferably less than about 2.5 wt/vol %, such as from about 0.01 to about 2.0 wt/vol % and more preferably from about 0.1 to about 1.5 wt/vol %. For example, in one embodiment, the composition comprises from about 0.1 to about 2.0 wt/vol % of a single therapeutic agent selected from the group consisting of maltitol, pullulan, maltotriose, 2-deoxy-D-ribose, 1-kestose and erlose.

Further, the therapeutic agent should be provided in an amount sufficient to provide a therapeutic effect when administered to a user. For example, the single therapeutic agent is present in an amount sufficient to stimulate the growth of certain healthy bacteria such as *Lactobacillus bulgaricus, Lactobacillus acidophilus, Lactobacillus gasseri, Lactobacillus crispatus, Lactobacillus casei, Lactobacillus plantarum.* Preferably the composition provides a therapeutic effect, measured as the ratio of *L. crispatus* to *E. coli* as described in the test methods section below, greater than about 30, more preferably greater than about 100, and more preferably greater than about 200, and even more preferably greater than about 300.

The compositions of the present invention may be formulated for administration to a user. For example, in those embodiments where the composition is formulated as a vaginal treatment formulation, it may be formulated as a spray, moisturizer, lotion, cream, jelly, liniment, ointment, salve, oil, foam, gel, film, wash, suppository, slow-releasing polymer, coating, liquid, vaginal capsule, vaginal tablet, vaginal film, vaginal sponge, vaginal ovule, etc. The composition may also be applied to a vaginal insert, tampon, wipe or pad, and then administered to the vagina. Formulations may contain a single therapeutic agent selected from the group consisting of maltitol, pullulan, maltotriose, 2-deoxy-D-ribose, 1-kestose and erlose, an aqueous solvent and optionally a dermatologically acceptable carrier. As used herein, "dermatologically acceptable carrier" generally refers to a carrier that is suitable for topical application to the keratinous tissue and is compatible with a prebiotic. The dermatologically acceptable carrier may be in a wide variety of forms such as, for example, simple solutions (water-based or oil-based), solid forms (e.g. gels or sticks) and emulsions.

The solvent is aqueous. Water is a particularly preferred aqueous solvent. Typically, the solvent constitutes greater than about 75 wt/vol %, more preferably greater than about 85 wt/vol %, and still more preferably greater than about 90 wt/vol %.

The compositions of the present invention are generally acidic, i.e., have a pH less than about 7.0 and more preferably less than about 6.0, such as from about 3.0 to about 6.0 and still more preferably from about 4.0 to about 5.0. In a particularly preferred embodiment the pH may be maintained at a mildly acidic level to correspond to normal vaginal conditions. For example, the pH may be within a range of from about 3.0 to about 6.0, in some embodiments from about 3.5 to about 5.0, and in some embodiments, from about 4.0 to about 4.5. The foregoing acid pH may also provide other benefits. For instance, when the composition is configured to form a gel, such as described below, a low pH level may also improve the gelation rate and gel strength to reduce the likelihood of leakage just after insertion of the composition into the vagina.

The pH of the composition may be adjusted using an organic acid. Organic acids useful in the present invention generally consist of mono- or polycarboxylic acids having one or more hydroxyl functional groups at least one of which is introduced into the α-position (i.e., on the carbon atom adjacent to the carboxyl functional group). Examples of particularly useful organic acids include citric acid, lactic acid, methyllactic acid, phenyllactic acid, malic acid, mandelic acid, glycolic acid, tartronic acid, tartaric acid and gluconic acid. In particularly preferred embodiments the organic acid is selected from the group consisting of citric acid, lactic acid, malic acid, glycolic acid and tartaric acid. In certain embodiments the organic acid may be provided with an appropriate counterion, such as calcium, sodium or magnesium. In particularly preferred embodiments the composition may comprise a single therapeutic agent selected from the group consisting of maltitol, pullulan, maltotriose, 2-deoxy-D-ribose, 1-kestose and erlose and an organic acid. In other embodiments the composition may comprise a single therapeutic agent selected from the group consisting of maltitol, pullulan, maltotriose, 2-deoxy-D-ribose, 1-kestose and erlose and an organic acid selected from the group consisting of citric acid, lactic acid, malic acid, glycolic acid and tartaric acid.

In view of the foregoing, in certain embodiments the compositions and formulations of the present invention may have a pH from about 3.0 to about 6.0, more preferably from about 3.5 to about 5.0, and comprise a single therapeutic agent selected from the group consisting of maltitol, pullulan, maltotriose, 2-deoxy-D-ribose, 1-kestose and erlose, wherein the total amount of therapeutic agent is from about 0.1 to about 2.0 wt/vol %.

In one particular embodiment of the present invention, for example, the composition is configured to rapidly form a gel when applied to the vagina. A "gel" is a colloid in which a disperse phase combines with a dispersion medium to produce a jelly-like, solid or semi-solid material. The gel may form in less than about one hour, in some embodiments less than about one minute, and in some embodiments, less than about 30 seconds. Among other things, such rapid gelation reduces the likelihood of leakage during use. In addition, because the gel may form intravaginally, it is more likely to retain its structure and shape over an extended period of time. In this manner, the gel may provide the prolonged release of a therapeutic agent that inhibits and/or treats vaginal infection. For instance, the gel may remain within the vagina for about 2 to about 48 hours to provide the desired effect.

Although a variety of compounds may be employed, water is usually employed as the dispersion medium for the gel to optimize biocompatibility. Other possible dispersion mediums include non-aqueous solvents, including glycols, such as propylene glycol, butylene glycol, triethylene glycol, hexylene glycol, polyethylene glycols, ethoxydiglycol, and dipropyleneglycol; alcohols, such as ethanol, n-propanol, and isopropanol; triglycerides; ethyl acetate; acetone; triacetin; and combinations thereof. Typically, the dispersion medium (e.g., water) constitutes greater than about 75 wt/vol %, in some embodiments greater than about 90 wt/vol %, and in some embodiments, from about 95 to about 99 wt/vol % of the composition.

The disperse phase of the gel may be formed from any of a variety of different gelling agents, including temperature responsive ("thermogelling") compounds, ion responsive compounds, and so forth. Thermogelling systems, for instance, respond to a change in temperature (e.g., increase in temperature) by changing from a liquid to a gel. Generally speaking, the temperature range of interest is from about 25°C to about 40°C, in some embodiments from about 35°C to about 39°C, and in one particular embodiment, at the human body temperature (about 37°C). Compositions that change state at about this temperature are useful because they will remain in a body cavity, for example, after they have been delivered. Any of a variety of thermogelling compounds that are capable of gelling when applied to the vagina may be used in the present invention. In some cases, thermogelling block copolymers, graft copolymers, and/or homopolymers may be employed. For example, polyoxyalkylene block copolymers may be used in some embodiments of the present invention to form a thermo-gelling composition. Suitable thermo-gelling compositions may include, for example, homopolymers, such as poly(N-methyl-N-n-propylacrylamide), poly(N-n-propylacrylamide), poly(N-methyl-N-isopropylacrylamide), poly(N-n-propylmethacrylamide), poly(N-isopropylacrylamide), poly(N,n-diethylacrylamide); poly(N-isopropylmethacrylamide), poly(N-cyclopropylacrylamide), poly(N-ethylmethyacrylamide), poly(N-methyl-N-ethylacrylamide), poly(N-cyclopropylmethacrylamide), and poly(N-ethylacrylamide). Still other examples of suitable thermogelling polymers may include cellulose ether derivatives, such as hydroxypropyl cellulose, methyl cellulose, hydroxypropylmethyl cellulose, and ethylhydroxyethyl cellulose. Moreover thermogelling polymers may be made by preparing copolymers between (among) monomers, or by combining such homopolymers with other water-soluble polymers, such as acrylic monomers (e.g., acrylic or methacrylic acid, acrylate or methacrylate, acrylamide or methacrylamide, and derivatives thereof).

The compositions of the present invention may also include an ion responsive compound. Such compounds are generally well known in the art, and tend to form a gel in the presence of certain ions or at a certain pH. For instance, one suitable class of ion responsive compounds that may be employed in the present invention is anionic polysaccharides. Anionic polysaccharides may form a three-dimensional polymer network that functions as the disperse phase of the gel. Generally speaking, anionic polysaccharides include polysaccharides having an overall anionic charge, as well as neutral polysaccharides that contain anionic functional groups.

Any of a variety of anionic polysaccharides capable of forming a gel when contacted with vaginal mucosa may be used in the present invention. Such gel-forming anionic polysaccharides are typically stable over the normal acidic pH values found in the vagina (e.g., from about 2.5 to about 5.5). For instance, some suitable examples of gel-forming anionic polysaccharides include natural gums, such as gellan gum and alginate gums (e.g., ammonium and alkali metal of salts of alginic acid); chitosan; carboxymethylcellulose, pectins, carrageenan, xantham gum, and derivatives or salts thereof. The particular type of anionic polysaccharide selected will depend, in part, on the nature of the composition and the other components used therein. For example, carrageenan is sensitive to particular types of cations, e.g., it typically gels in the presence of potassium but not sodium. Glycuronans, likewise, typically gel in the presence of divalent cations (e.g., Ca²⁺), but not monovalent cations (e.g., Na+). Xanthan gum may gel in the presence of divalent cations, but only at a relatively high pH.

Although any of the above-described anionic polysaccharides may be used in the present invention, gellan gum is particularly desired for use in the present invention, either alone or in combination with other gelling agents, because it is able to form a gel in the presence of a wide variety of different cations, including both monovalent and divalent cations. Gellan gum is intended to encompass any form of gellan, including native gellan, clarified gellan, deacylated gellan, nonacylated gellan (e.g., produced from genetically engineered bacteria), clarified gellan (the polysaccharide is fully or partially removed from the bacterial debris), chemically modified gellan, etc. Various types of gellan gums and methods for forming such gums are described in U.S. Pat. Nos. 4,326,052, 4,326,053, 4,377,636, 4,385,123, and 4,563,366. Suitable gellan gums are commercially available from a variety of different sources. For example, GELRITE^{™} gellan gum is available from Sigma-Aldrich Chemical Co. of St. Louis, MO, and is produced from a naturally occurring polysaccharide after deacylation and clarification. Deacylated gellan is also available from CP Kelco U.S., Inc. of Chicago, IL under the name KELCOGEL^{®}.

Gellan gum may be either high or low acyl gellan. In the high acyl (or "native") form, two acyl substituents, acetate and glycerate, are present. Both substituents are located on the same glucose residue and, on average, there is one glycerate per repeat unit and one acetate per every two repeat units. In the low acyl form, the acyl groups may be wholly or partially removed through deacylation. The degree of deacylation of deacylated gellan gums may be at least about 20%, in some embodiments at least about 50%, and in some embodiments, at least about 75%. Alternatively, the low acyl gellan gum may simply be "nonacylated" in that it is formed without acyl groups by genetically engineered bacteria. Regardless of the manner in which they are formed, low acyl gellan gums generally have a gelation temperature within the range 30 to 50°C, which may be particularly well suited for use in the present invention so that it may gel at body temperatures of about 37°C, but remain stable at typical storage and transportation temperatures of about 25°C. In addition, low acyl gellan gums are also firm and elastic, and thus may retain their shape after delivery to the vaginal cavity.

In most embodiments the gelling agent(s) are present in an amount of from about 0.01 to about 10.0 wt/vol %, in some embodiments from about 0.05 to about 5.0 wt/vol %, and in some embodiments, from about 0.1 to about 1.0 wt/vol % of the composition.

If desired, a gelling composition may be provided in any desired form (e.g., liquid, powder, etc.). In fact, one particular benefit of the composition is that it may be administered as a liquid, which allows for the selection of a wider variety of administration techniques than would otherwise be available for a solid or semi-solid gel. One technique that may be employed includes dispensing the composition through a liquid applicator, such as a syringe or tube, into the vaginal cavity. The administered volume of the composition may constitute a single dose or two or more doses. Although not necessarily required, the composition of may also be sterilized prior to administration. Sterilization may be accomplished by any technique known in the art, such as using a gas (e.g., ethylene oxide), radiation (e.g., gamma), or heat (autoclaving). If desired, the composition may be subjected to one or more filtration steps prior to sterilization to help remove contaminants.

The compositions of the present invention may be applied to a suitable substrate, which in-turn may be used to apply the therapeutic agent to a user. Suitable applicators include a web, such as a wet laid tissue web or air laid web, gauze, cotton swab, transdermal patch, container or holder. Particularly preferred applicators include fibrous webs, including flushable and non-flushable cellulosic webs and nonwoven webs of synthetic fibrous material. Useful webs may be wet laid, air laid, meltblown, or spunbonded. Suitable synthetic fibrous material includes meltblown polyethylene, polypropylene, copolymers of polyethylene and polypropylene, bicomponent fibers including polyethylene or polypropylene, and the like. Useful nonwoven webs may be meltblown, coform, spunbond, airlaid, hydroentangled nonwovens, spunlace, bonded carded webs.

In certain embodiments, particularly those in which the composition is applied to a web, it may be desirable that the formulation provide certain physical attributes, such as having a smooth, lubricious, non-greasy feel; the ability to at least partially transfer from the web to the user's skin; the capability to be retained on the web at about room temperature; or the ability to be compatible with the web manufacturing process. In certain embodiments it is preferred that at least a portion of the composition is transferred from the tissue to the user's skin in use.

The composition may be applied to a web during formation of the web or after the web has been formed and dried, often referred to as off-line or post-treatment. Suitable methods of applying the composition to a web include methods known in the art such as gravure printing, flexographic printing, spraying, WEKO^{™}, slot die coating, or electrostatic spraying. One particularly preferred method of off-line application is rotogravure printing.

### TEST METHODS

### Therapeutic Effect Protocol

Colonies of *L. crispatus* and *E. coli* were prepared as follows. A colony of *L. crispatus* was transferred to 7ml MRS broth and incubated anaerobically (using BD GasPak EZ anaerobe container system with indicator) at 37°C without shaking for 18-20 hours. A colony of *E. coli* was transferred to 5ml TSB broth and incubated aerobically (shaking at 100rpm) at 37°C for 18-20 hours.

Colonies were then inoculated as follows. Bacterial cultures were gently vortexed and 1 mL of each culture was transferred to a corresponding 2.0 mL micro-centrifuge tube and then centrifuged for two minutes at 14,500 rpm. The cultural supernatant was removed and the cell pellet was re-suspended in 1 mL of 0.95% (wt/vol %) saline. The re-suspended colony was then centrifuged for two minutes at 14,500 rpms and the supernatant was removed. For *L. crispatus,* the pellet was re-suspended in 1 mL of 0.95% saline to achieve ~10⁷ - 10⁸ cfu/mL. For *E. coli* the pellet was re-suspended in 1 mL of 0.95% saline to achieve ~10⁸ - 10⁹ cfu/mL.

Media were prepared as follows.

**TABLE 1**

| LAPT-g Media Ingredients | | |
|---|---|---|
| Ingredients | g/L | g/L |
| | 1X | 2X |
| Peptone | 15 | 30 |
| Tryptone | 10 | 20 |
| Yeast Extract | 10 | 20 |
| Tween 80 | 1 | 2 |

All of the ingredients in Table 1 were combined and the pH was adjusted to 6.5. The media were then autoclaved for 20 minutes at 125°C. To evaluate the effect of various saccharides and organic acids (noted as carbon sources below), the following samples were prepared:

**TABLE 2**

| Carbon Testing Media | |
|---|---|
| Ingredient | mL |
| 2x LAPT-g Media | 50 |
| 20% (wt/vol %) Carbon Source (filter sterilized) | 5 |
| Sterile H₂O | 45 |
| Total | 100 |

Five milliliters (5 mL) of each medium was transferred to a test tube in duplicates for subsequent inoculation. A master mixture of *L. crispatus* and *E. coli,* prepared as described above, was prepared in 1,000:1 ratio. Each (5ml) test tube was inoculated with the master mixture to give 10⁵ - 10⁶ total CFU *L. crispatus* and 100-1,000 total CFU *E. coli* per tube.

To establish a negative control, one tube was vortexed and 100 µL was removed to determine the initial cell concentrations by serial dilutions and plating (2 plates per dilution). *L. crispatus* is selected on MRS agar plates incubated anaerobically at 37°C for two days. *E. coli* is selected on TSA plates incubated aerobically at 37°C for one day. The co-cultures were incubated in an anaerobic container with BD GasPaks at 37°C for 36 hours.

The effect of the carbon source on the ratio of *L. crispatus* to *E. coli* was measured 36 hours after inoculation. The co-culture tube was vortexed and 100 µL was removed to determine the final cell concentrations by serial dilutions and plating (2 plates per dilution). *L. crispatus* is selected on MRS agar plates incubated anaerobically at 37°C for two days. *E. coli* is selected on TSA plates incubated aerobically at 37°C for one day.

### EXAMPLES

Inventive samples were prepared by adding a carbon source as set forth in Table 3 below. The therapeutic effect of the formulation was then measured using the assay described above in the Test Methods section above. The therapeutic effect is summarized below as the ratio of *L. crispatus* to *E. coli.*

**TABLE 3**

| Sample | Therapeutic Agent | CAS # | Concentration (wt/vol %) | *L. crispatus*/*E. coli* ratio |
|---|---|---|---|---|
| 1 | 1-Kestose | 470-69-9 | 1 | 640 |
| 2 | 2-Deoxy-D-Ribose | 533-67-5 | 1 | 161 |
| 3 | Erlose | 13101-54-7 | 1 | 32.1 |
| 4 | Maltitol | 585-88-6 | 1 | 202 |
| 5 | Maltotriose | 1109-28-0 | 1 | 535 |
| 6 | Palatinose | 13718-94-0 | 1 | 600 |
| 7 | Pullulan | 9057-02-7 | 1 | 248 |

## Claims

1. A composition for use in maintaining a healthy microflora balance in the urogenital area of a patient in need thereof by topically administering the composition to the urogenital area of a patient, the composition comprising a single therapeutic agent selected from the group consisting of maltitol, pullulan, maltotriose, 2-deoxy-D-ribose, 1-kestose and erlose and an aqueous solvent, wherein the urogenital area is the vulva, vagina, or urinary tract.

2. The composition for use according to claim 1 wherein the single therapeutic agent comprises from 0.1 to 2.0 wt/vol % of the composition.

3. The composition for use according to claim 1 wherein the composition has a pH from 3.0 to 5.0.

4. The composition for use according to claim 1 wherein administration of the composition maintains the pH of the urogenital area in the range from 3.5 to 4.5.

5. The composition for use according to claim 1 wherein the single therapeutic agent is the sole source of carbon for bacterium administered to the patient.

6. The composition for use according to claim 1 wherein the composition further comprises an organic acid selected from the group consisting of citric acid, lactic acid, methyllactic acid, phenyllactic acid, malic acid, mandelic acid, glycolic acid, tartronic acid, tartaric acid and gluconic acid.

7. The composition for use according to claim 1 wherein the single therapeutic agent is selected from the group consisting of maltitol, pullulan, maltotriose, 2-deoxy-D-ribose and 1-kestose.

8. The composition for use according to claim 1 wherein the single therapeutic agent is 1-kestose.

## Patentansprüche

1. Zusammensetzung zur Verwendung bei der Aufrechterhaltung eines gesunden Mikroflora-Gleichgewichts im Urogenitalbereich eines Patienten, der dieser bedarf, durch topische Verabreichung der Zusammensetzung an den Urogenitalbereich eines Patienten, wobei die Zusammensetzung einen therapeutischen Einzelwirkstoff, ausgewählt aus der Gruppe, bestehend aus Maltitol, Pullulan, Maltotriose, 2-Desoxy-D-ribose, 1-Kestose und Erlose, und ein wässriges Lösungsmittel umfasst, wobei der Urogenitalbereich die Vulva, die Vagina oder der Harntrakt ist.

2. Zusammensetzung zur Verwendung nach Anspruch 1, wobei der therapeutische Einzelwirkstoff von 0,1 bis 2,0 Gew./Vol.-% der Zusammensetzung umfasst.

3. Zusammensetzung zur Verwendung nach Anspruch 1, wobei die Zusammensetzung einen pH-Wert von 3,0 bis 5,0 aufweist.

4. Zusammensetzung zur Verwendung nach Anspruch 1, wobei die Verabreichung der Zusammensetzung den pH-Wert des Urogenitalbereichs im Bereich von 3,5 bis 4,5 aufrechterhält.

5. Zusammensetzung zur Verwendung nach Anspruch 1, wobei der therapeutische Einzelwirkstoff die alleinige Kohlenstoffquelle für das dem Patienten verabreichte Bakterium ist.

6. Zusammensetzung zur Verwendung nach Anspruch 1, wobei die Zusammensetzung ferner eine organische Säure umfasst, ausgewählt aus der Gruppe, bestehend aus Citronensäure, Milchsäure, Methylmilchsäure, Phenylmilchsäure, Äpfelsäure, Mandelsäure, Glykolsäure, Tartronsäure, Weinsäure und Gluconsäure.

7. Zusammensetzung zur Verwendung nach Anspruch 1, wobei der therapeutische Einzelwirkstoff aus der Gruppe ausgewählt ist, bestehend aus Maltitol, Pullulan, Maltotriose, 2-Desoxy-D-ribose und 1-Kestose.

8. Zusammensetzung zur Verwendung nach Anspruch 1, wobei der therapeutische Einzelwirkstoff 1-Kestose ist.

## Revendications

1. Composition pour utilisation dans le maintien d'un équilibre sain de la microflore dans la zone urogénitale d'un patient en ayant besoin par administration topique de la composition à la zone urogénitale d'un patient, la composition comprenant un agent thérapeutique unique choisi dans le groupe constitué de maltitol, pullulane, maltotriose, 2-désoxy-D-ribose, 1-kestose et erlose et un solvant aqueux, dans laquelle la zone urogénitale est la vulve, le vagin ou les voies urinaires.

2. Composition pour utilisation selon la revendication 1, dans laquelle l'unique agent thérapeutique comprend de 0,1 à 2,0 % en poids/volume de la composition.

3. Composition pour utilisation selon la revendication 1, dans laquelle la composition a un pH de 3,0 à 5,0.

4. Composition pour utilisation selon la revendication 1, dans laquelle l'administration de la composition maintient le pH de la zone urogénitale dans la plage de 3,5 à 4,5.

5. Composition pour utilisation selon la revendication 1, dans laquelle l'unique agent thérapeutique est la seule source de carbone pour la bactérie administrée au patient.

6. Composition pour utilisation selon la revendication 1, dans laquelle la composition comprend en outre un acide organique choisi dans le groupe constitué d'acide citrique, acide lactique, acide méthyllactique, acide phényllactique, acide malique, acide mandélique, acide glycolique, acide tartronique, acide tartrique et acide gluconique.

7. Composition pour utilisation selon la revendication 1, dans laquelle l'unique agent thérapeutique est choisi dans le groupe constitué de maltitol, pullulane, maltotriose, 2-désoxy-D-ribose et 1-kestose.

8. Composition pour utilisation selon la revendication 1, dans laquelle l'unique agent thérapeutique est le 1-kestose.
